(19) Europäisches Patentamt
European Patent Office
Office européen des brevets

(11) EP 0 940 386 B1

(12) FASCICULE DE BREVET EUROPEEN

(45) Date de publication et mention de la délivrance du brevet:
**22.05.2002 Bulletin 2002/21**

(51) Int Cl.[7]: **C07C 215/42**, C07C 217/74, C07D 309/10, C07D 335/02, C07D 233/48, C07D 263/28, A61K 31/35

(21) Numéro de dépôt: **99400506.4**

(22) Date de dépôt: **03.03.1999**

# (54) Nouveaux composés du benzocyclobutane, leur procédé de préparation et les compositions pharmaceutiques qui les contiennent

Benzozyclobutanverbindungen, deren Herstellungsverfahren und diese enthaltende pharmazeutische Zusammenstellungen

Benzocyclobutan compounds, their process for preparation and pharmaceutical compositions containing them

(84) Etats contractants désignés:
**AT BE CH DE DK ES FI FR GB GR IE IT LI LU NL PT SE**

(30) Priorité: **04.03.1998 FR 9802585**

(43) Date de publication de la demande:
**08.09.1999 Bulletin 1999/36**

(73) Titulaire: **LES LABORATOIRES SERVIER**
**92200 Neuilly sur Seine (FR)**

(72) Inventeurs:
- **Peglion, Jean-Louis**
**78110 Le Vesinet (FR)**
- **Dessinges, Aimée**
**92500 Rueil Malmaison (FR)**
- **Harmange, Jean-Christophe**
**Andover MA 01810 (US)**
- **Millan, Mark**
**78230 Le Pecq (FR)**
- **Newman-Tancredi, Adrian**
**78230 Le Pecq (FR)**
- **Brocco, Mauricette**
**75003 Paris (FR)**

(56) Documents cités:
**EP-A- 0 457 686**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 940 386 B1

## Description

La présente invention a pour objet de nouveaux composés du benzocyclobutane, leur procédé de préparation et les compositions pharmaceutiques qui les contiennent.

Les composés de la présente invention agissent comme de puissants inhibiteurs de recapture de sérotonine et comme des inhibiteurs de la recapture de noradrénaline.

A ce titre ils peuvent être utilisés en thérapeutique pour le traitement de la dépression, des attaques de panique, des troubles obsessionnels compulsifs, des phobies, des troubles impulsifs, de l'abus de drogue et de l'anxiété.

En effet, dans le test de la nage forcée chez la souris (Porsolt et al., *Arch. Intern. Pharmacodyn. Therap.*, **1977,** 229, p 327) et dans le test de suspension par la queue chez la souris (Stéru et al., *Psychopharmacology*, **1985,** 85, 367-370) les produits de la présente invention montrent une excellente activité en comparaison des produits de référence comme la fluoxétine.

L'état antérieur de la technique le plus proche de la présente invention est illustré par le brevet EP 0 457 686 B1 qui concerne des antagonistes des récepteurs $5HT_{1A}$ utilisables dans le traitement de la douleur, du stress, de la migraine, de l'anxiété, de la dépression et de la schizophrénie, dont la structure ne saurait toutefois induire celle des composés de la présente invention.

Plus spécifiquement, la présente invention concernent les composés de formule (I) :

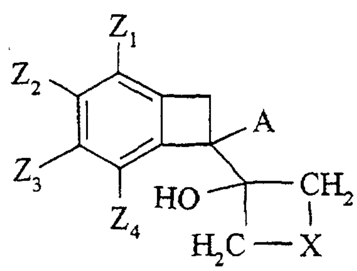

(I)

dans laquelle :

$Z_1$, $Z_2$, $Z_3$ et $Z_4$, identiques ou différents, représentent chacun un atome d'hydrogène, d'halogène, un groupement ($C_1$-$C_6$)alkyle linéaire ou ramifié, ($C_2$-$C_6$)alkényle linéaire ou ramifié, ($C_2$-$C_6$)alkynyle linéaire ou ramifié, [($C_3$-$C_8$) cycloalkyl]-($C_1$-$C_6$) alkyle, la partie alkyle étant linéaire ou ramifié, hydroxy, ($C_1$-$C_6$)alkoxy linéaire ou ramifié, benzyloxy ($C_2$-$C_6$)alkénoxy linéaire ou ramifié, ($C_2$-$C_6$)alkynoxy linéaire ou ramifié, cyano, trifluorométhyle, trifluorométhoxy, nitro, un groupement de formule $-OSO_2CF_3$, $OSO_2CH_3$, $-NHCOCH_3$, $-NHCOCF_3$, $-NHSO_2CH_3$,

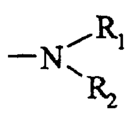

ou un groupement de formule

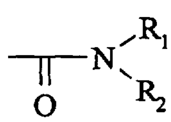

dans lesquelles

$R_1$ et $R_2$, identiques ou différents, représentent chacun un atome d'hydrogène, un groupement ($C_1$-$C_6$)alkyle linéaire ou ramifié, ($C_2$-$C_6$)alkényle linéaire ou ramifié, ($C_2$-$C_6$)alkynyle linéaire ou ramifié, arylalkyl ($C_1$-$C_6$) linéaire ou ramifié ou [($C_3$-$C_8$)cycloalkyl]-($C_1$-$C_6$)alkyle, la partie alkyle étant linéaire ou ramifié,

X représente :

- un atome d'oxygène,
- un groupement de formule $S(O)_p$ dans lequel p représente un entier compris entre 0 et 2 inclus,
- un groupement de formule $-(CH_2)_n$ dans lequel n représente un entier compris entre 1 et 4 inclus,
- ou un groupement de formule $-CH_2-Y-CH_2-$ dans lequel Y représente un atome d'oxygène, un atome de selenium, un groupement $>S(O)_p$ avec p tel que défini précédemment, $>N-R_1$ ou

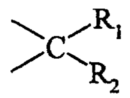

dans lesquels $R_1$ et $R_2$ ont la même signification que précédemment,

A représente un groupement de formule

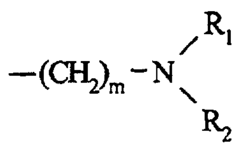

ou

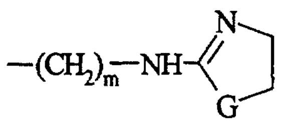

dans lesquels

$R_1$ et $R_2$ ont la signification précédemment définie,
m représente un nombre entier compris entre 1 et 6 inclus, et
G représente un atome d'oxygène ou le groupe NH,

leurs isomères ainsi que leurs sels d'addition à un acide pharmaceutiquement acceptable.

Par groupement aryle, on entend un groupement phényle, naphtyle, indène, tétrahydronaphtyle, dihydronaphtyle, dihydroindène, chacun de ces groupements étant éventuellement substitué de façon identique ou différente, par un ou plusieurs groupements choisis parmi atome d'halogène, groupement alkyle ($C_1$-$C_6$) linéaire ou ramifié, hydroxy ou alkoxy ($C_1$-$C_6$) linéaire ou ramifié.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Le groupement A préféré des composés de l'invention est le groupement de formule

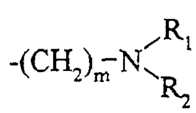

dans lequel m, $R_1$ et $R_2$ sont tels que définis précédemment.

Selon une variante avantageuse de l'invention, les composés préférés sont les composés de formule (I) dans laquelle X représente un groupement de formule $-(CH_2)_n-$ dans lequel n est tel que défini dans la formule (I).

D'une façon particulièrement avantageuse, les composés préférés de l'invention sont les composés de formule (I) dans laquelle :

- A représente un groupement de formule

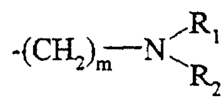

avec $R_1$ et $R_2$ tels que définis précédemment et m est égal à 1,
- et X représente un groupement de formule $-(CH_2)_n-$ avec n est égal à 3.

Selon une autre variante de l'invention, les composés préférés sont les composés de formule (I) dans laquelle X représente un groupement de formule $-CH_2-Y-CH_2$ dans laquelle Y est tel que défini dans la formule (I).
Les groupements $Z_1$, $Z_2$, $Z_3$ et $Z_4$ préférés, identiques ou différents, sont les groupements prenant les définitions choisies parmi atome d'hydrogène, d'halogène, groupement hydroxy, alcoxy ($C_1$-$C_6$) linéaire ou ramifié, alkyle ($C_1$-$C_6$) linéaire ou ramifié, trifluorométhyle et trifluorométhoxy.
Les composés préférés de l'invention sont les composés de formule (I) correspondant aux :

* 1 -(N,N-diméthylaminométhyl)-1-(1-hydroxycyclohex-1-yl)-5-méthoxybenzocyclobutane,
* 1-(N,N-diméthylaminométhyl)-1-(1-hydroxycyclohex-1-yl)-4-méthoxybenzocyclobutane,
* et (-)1-(N,N-diméthylaminoéthyl)-1-(1-hydroxycyclohex-1-yl)-5-méthoxybenzocyclobutane.

Les isomères ainsi que les sels d'addition à un acide pharmaceutiquement acceptable des composés préférés font partie intégrante de l'invention.
La présente invention a également pour objet le procédé de préparation des composés de formule (I) caractérisé en ce que l'on utilise comme produit de départ :

* *soit un composé de formule (II) :*

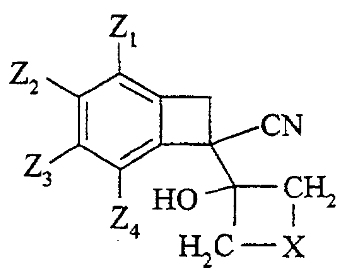

(II)

dans laquelle $Z_1$, $Z_2$, $Z_3$, $Z_4$ et X sont tels que définis dans la formule (I),
que l'on traite par un hydrure dans l'éther ou le tétrahydrofurane, pour conduire aux composés de formule (I/a), cas particulier des composés de formule (I):

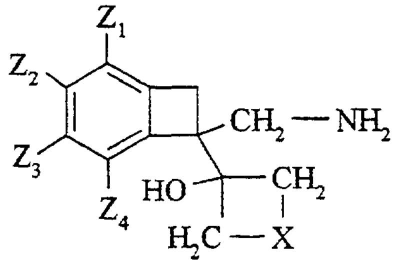

(I/a)

dans laquelle $Z_1$, $Z_2$, $Z_3$, $Z_4$ et X ont les significations précédemment définies,
composés de formule (Va) que l'on substitue en utilisant les méthodes classiques de la chimie organique telles que par exemple :

- amination réductrice à partir des aldéhydes ou cétones correspondants, en présence de cyanoborohydrure de sodium ou de triacétoxyborohydrure de sodium, ou
- substitution nucléophile de dérivés de formule (III) :

$$R'_1Z \qquad (III)$$

dans laquelle $R'_1$ prend la signification précédemment définie pour $R_1$ à l'exception de la valeur hydrogène, et Z est un groupe partant tel que, par exemple I, Br, Cl,

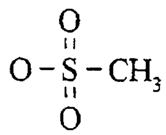

ou

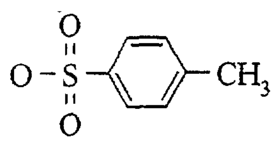

pour conduire aux amines secondaires de formule (I/b), cas particulier des composés de formule (I):

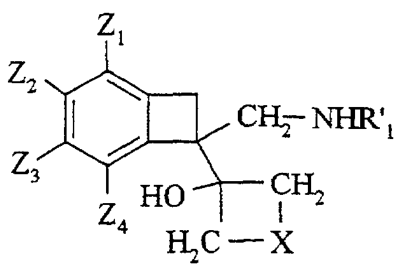

(I/b)

dans laquelle $Z_1$, $Z_2$, $Z_3$, $Z_4$, X et $R'_1$ ont les significations précédemment définies, composés de formule (I/b) qui sont à nouveau substitués par les mêmes méthodes que celles décrites ci-dessus pour conduire aux amines tertiaires de formule (Vc), cas particulier des composés (I):

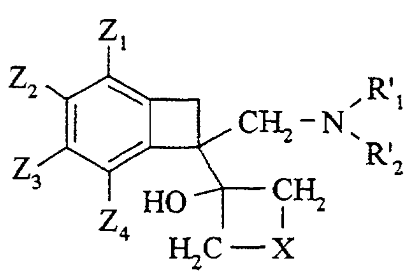

(I/c)

dans laquelle $Z_1$, $Z_2$, $Z_3$, $Z_4$ et X prennent les valeurs précédemment définies et $R'_1$ et $R'_2$ ont respectivement les significations précédemment définies pour $R_1$ et $R_2$ à l'exception de la valeur hydrogène, étant entendu que lorsque $R'_1$ et $R'_2$ sont identiques tout en étant chacun différent d'hydrogène, les amines tertiaires de formule (I/c) sont obtenues directement à partir de l'amine (I/a) sans avoir à isoler l'amine secondaire (I/b),

* *soit un composé de formule (IV)* :

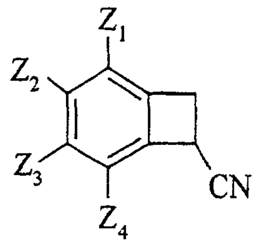

(IV)

dans laquelle $Z_1$, $Z_2$, $Z_3$, $Z_4$ sont tels que définis dans la formule (I), que l'on substitue, en présence d'une base forte, par une amine de formule (V) :

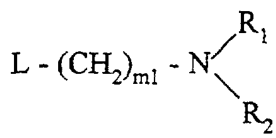

(V)

dans laquelle $R_1$ et $R_2$ sont tels que définis dans la formule (I), ml est un entier compris entre 2 et 6 inclus et L représente un groupe partant tel qu'un atome d'halogène, un groupement mésylate, tosylate, trifluorométhanesulfonate, etc...
pour conduire aux composés de formule (VI) :

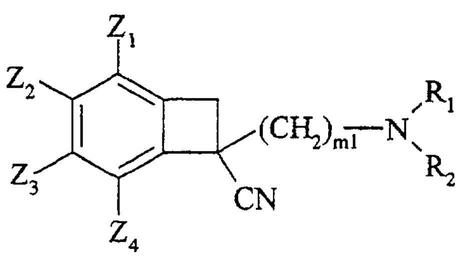

(VI)

dans laquelle $Z_1$, $Z_2$, $Z_3$, $Z_4$, $R_1$, $R_2$ et m1 sont tels que définis précédemment, composés de formule (VI) que l'on traite dans des conditions d'hydrolyse alcoolique acide en présence d'un composé de formule (VII) :

G - OH (VII)

dans laquelle G représente un groupement alkyle ($C_1$-$C_6$) linéaire ou ramifié, pour conduire aux composés de formule (VIII):

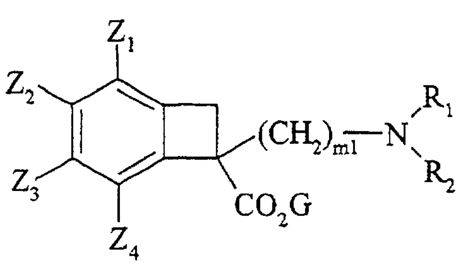

(VIII)

dans laquelle $Z_1$, $Z_2$, $Z_3$, $Z_4$, $R_1$, $R_2$, G et m1 sont tels que définis précédemment, composés de formule (VIII) que l'on traite par un dimagnésien de formule (IX) :

$$Z'\text{-}Mg\text{-}CH_2\text{-}X\text{-}CH_2\text{-}Mg\text{-}Z' \quad (IX)$$

dans laquelle X est tel que défini dans la formule (I) et Z' représente un atome d'halogène, pour conduire aux composés de formule (I/d), cas particulier des composés de formule (I) :

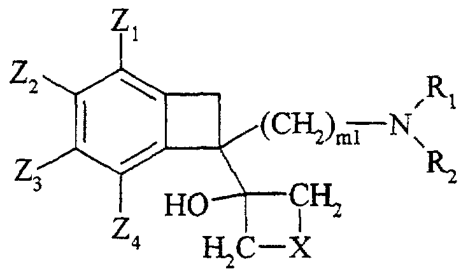

(I/d)

dans laquelle $Z_1$, $Z_2$, $Z_3$, $Z_4$, $R_1$, $R_2$, X et $m_1$ sont tels que définis précédemment, l'ensemble des composés de formule (I/a) et de formule (Vd), dans le cas particulier où $R_1$ et $R_2$ représentent simultanément un atome d'hydrogène, formant les composés de formule (I/e) :

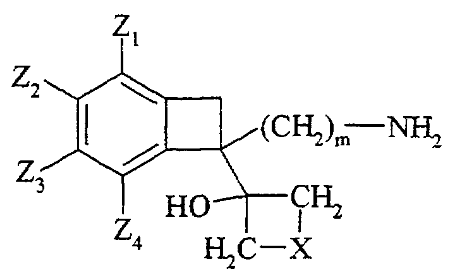

(I/e)

dans laquelle $Z_1$, $Z_2$, $Z_3$, $Z_4$, X et m sont tels que définis dans la formule (I), composés de formule (I/e) qui sont :

→ *soit traités par l'isocyanate de 2-chloroéthyle* dans l'acétonitrile en présence de triéthylamine pour conduire aux composés de formule (I/f), cas particulier des composés de formule (I):

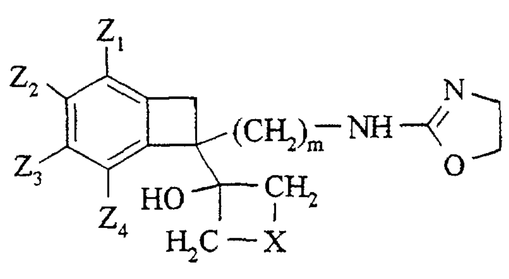

(I/f)

dans laquelle $Z_1$, $Z_2$, $Z_3$, $Z_4$, X et m ont les significations précédemment définies,

→ *soit traités par l'acide imidazolin-2-yl sulfonique* en présence d'une base organique ou minérale dans un alcool ou l'acétonitrile pour conduire aux composés de formule (I/g), cas particulier des composés de formule (I) :

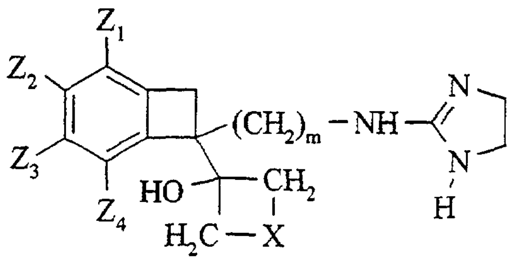

(I/g)

dans laquelle $Z_1$, $Z_2$, $Z_3$, $Z_4$, X et m ont les significations précédemment définies,

les composés (I/a) à (I/g) formant l'ensemble des composés de l'invention, que l'on purifie le cas échéant, selon une technique classique de purification, qui peuvent, si on le désire, être séparés en leurs différents isomères selon une technique classique de séparation, et que l'on transforme, le cas échéant, en leurs sels d'addition à un acide pharmaceutiquement acceptable.

Les matières premières de formules II et IV sont, soit des produits connus, soit des produits obtenus à partir de substances connues, selon des procédés connus.

La présente invention a également pour objet les compositions pharmaceutiques renfermant comme principe actif au moins un composé de formule (I), ses isomères optiques ou un de ses sels d'addition à un acide pharmaceutiquement acceptable, seul ou en combinaison avec un ou plusieurs excipients ou véhicules inertes, non toxiques et pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés sublinguaux, les sachets, les gélules, les tablettes, les suppositoires, les crèmes, les pommades, les gels dermiques, les préparations injectables ou buvables, les aérosols, les gouttes oculaires ou nasales, etc....

La posologie utile varie selon l'âge et le poids du patient, la voie d'administration, la nature et la sévérité de l'affection, et la prise de traitements éventuels et s'échelonne de 0,5 à 25 mg de principe actif, une à trois fois par jour.

Les exemples suivants, donnés à titre non limitatif, illustrent la présente invention.

Les points de fusion ont été déterminés soit à la platine chauffante de Kofler (K), soit à la platine chauffante sous microscope (MK).

Les produits de départ utilisés sont des produits connus ou préparés selon des modes opératoires connus.

**EXEMPLE 1**

**1-(N,N-diméthylaminométhyl)-1-(1-hydroxycyclohex-1-yl) benzocyclobutane et son chlorhydrate**

*Stade A : 1-cyano-1-(1-hydroxycyclohex-1-yl) benzocyclobutane*

En maintenant la température à - 78° C, on additionne 69 ml de butyllithium 1,6 M dans le n-hexane sur une solution de 12,9 g de 1-cyanobenzocyclobutane dans 200 ml de tétrahydrofurane. Après une demi-heure d'agitation à cette température, 15,5 ml de cyclohexanone sont additionnés et le mélange réactionnel est ramené à température ambiante. Après 3 heures d'agitation à température ambiante, le milieu réactionnel est hydrolysé à - 10° C par de l'eau. Après extraction par de l'éther éthylique, la phase organique est lavée par de la saumure, séchée sur $MgSO_4$ et concentrée sous vide. Le résidu cristallin est recristallisé de l'éther isopropylique pour donner 12,32 g du produit désiré

*Point de fusion (K): 109-110°C*

*Stade B : 1-aminométhyl 1-(1-hydroxycyclohex-1-yl) benzocyclobutane*

0,7 g (18,44 mM) de $LiAlH_4$ sont mis en suspension dans 38 ml d'éther. La suspension est refroidie à 0° C, puis on coule une solution de 1,72 g (7,58 mM) de produit obtenu au stade A dans 38 ml de tétrahydrofurane, en maintenant la température entre 0 et 5° C. Après une demi-heure à cette température le milieu réactionnel est agité 3 heures à température ambiante puis hydrolysé par 1 ml d'eau, 3 ml de soude à 20 % et encore 4 ml d'eau. On filtre et concentre pour obtenir 1,74 g de produit attendu.

*Stade C : Chlorhydrate du 1-(N,N-diméthylaminométhyl)-1-(1-hydroxycyclohex-1-yl) benzocyclobutane*

0,5 g (2,16 mM) de produit obtenu au stade B sont mis en solution dans 40 ml de $CH_3CN$. La suspension est refroidie à 0° C. On ajoute alors 0,3 g (4,33 mM) de cyanoborohydrure de sodium, et 1 ml (11 mM) de formol à 37 % dans l'eau. On agite 2 heures à température ambiante puis on rajoute les mêmes quantités de cyanoborohydrure de sodium et de formol et laisse encore agiter 18 heures à température ambiante. On hydrolyse par 5 ml d'HCl 1N et après 1 heure de contact on dilue par de l'eau distillée, lave à l'éther et basifie par de la soude normale. Après extraction, séchage et évaporation on obtient 0,5 g de produit désiré qui est transformé en son chlorhydrate par addition d'une solution d'éther chlorhydrique. Après recristallisation de l'acétonitrile on obtient 0,45 g du produit désiré.
*Point de fusion (M.K) : 234-235°C*

**EXEMPLE 2**

**1-(N,N-diméthylaminométhyl)-1-(1-hydroxycyclohex-1-yl)-4,5-diméthoxybenzocyclobutane et son chlorhydrate**

On procède comme dans l'exemple 1 en utilisant comme substrat au stade A le 1-cyano-4,5-diméthoxybenzocyclobutane.
*Point de fusion (M.K.) : 201-203°C*

**EXEMPLE 3**

**1-(N,N-diméthylaminométhyl)-1-(1-hydroxycyclohex-1-yl)-5-méthoxybenzocyclobutane et son chlorhydrate**

On procède comme dans l'exemple 1 en utilisant comme substrat au stade A le 1-cyano 5-méthoxybenzocyclobutane.
*Point de fusion (M.K): 225-227°C*

**EXEMPLE 4**

**1-(N,N-diméthylaminométhyl)-1-(1-hydroxycyclohex-1-yl)-4-méthoxybenzocyclobutane et son chlorhydrate**

On procède comme dans l'exemple 1 en utilisant comme substrat au stade A le 1-cyano-4-méthoxybenzocyclobutane.
*Point de fusion (M.K.) : 195-199°C*

**EXEMPLE 5**

**1-(aminométhyl)-1-(1-hydroxycyclohex-1-yl)-4-méthoxybenzocyclobutane et son chlorhydrate**

On procède comme dans l'exemple 1 des stades A à B en utilisant comme substrat au stade A le 1-cyano-4-méthoxybenzocyclobutane.
*Point de fusion (M.K.) : 206-209°C*

**EXEMPLE 6**

**1-(N-méthylaminométhyl)-1-(1-hydroxycyclohex-1-yl)-5-méthoxybenzocyclobutane et son chlorhydrate**

*Stade D : 1-(aminométhyl)-1-(1-hydroxycyclohex-1-yl)-5-méthoxybenzocyclobutane*

On procède comme au stade B de l'exemple 1 en utilisant comme produit de départ le 1-cyano 1-(1-hydroxycyclohex-1-yl)-5-méthoxybenzocyclobutane.

*Stade E : N-{(1-hydroxycyclohex-1-yl 5-méthoxybenzocyclobutane-1-yl)méthyl} carbamate d'éthyle*

Une solution de 2 g (7,65 mM) du composé obtenu au stade D et de 2,6 ml (19,8 mM) de triéthylamine dans 100 ml de chlorure de méthylène est coulée sur une solution refroidie à 0°C, de 0,61 ml (7,65 mM) de chloroformiate d'éthyle dans 50ml de chlorure de méthylène. On laisse agiter 24 heures à température ambiante, dilue à l'eau, extrait

au chlorure de méthylène. Les phases organiques sont d'abord lavées avec HCl 1N, puis à l'eau jusqu'à neutralité et enfin séchées sur $MgSO_4$ pour conduire après évaporation à 2,2 g de produit attendu.

*Stade F : Chlorhydrate du 1-(N-méthylaminométhyl)-1-(1-hydroxycyclohex-1-yl)-5-méthoxybenzocyclobutane*

Une solution de 2,2 g (6,6 mM) du produit obtenu au stade E dans 50 ml de tétrahydrofurane est introduite goutte à goutte dans une suspension de 376 mg de $LiAlH_4$ (9,9 mM) dans 40 ml de tétrahydrofurane. On porte 3 heures à reflux, puis on abandonne la nuit à température ambiante. Après hydrolyse par 0,26 ml d'eau, 0,2 ml de soude et 0,94 ml d'eau, on filtre et évapore à sec pour obtenir 1,8 g de produit attendu que l'on transforme en chlorhydrate par addition d'éther chlorhydrique.
*Point de fusion (M.K.) : 199-206°C*

**EXEMPLE 7**

**Isomère (+) du 1-(N,N-diméthylaminométhyl)-1-(1-hydroxycyclohex-1-yl)-5-méthoxybenzo cyclobutane et son chlorhydrate**

2 g du composé de l'exemple 3 sont soumis à une chromatographie à haute performance (H.P.L.C.) sur une colonne chirale de type CHIRALCEL OD®, la phase mobile étant composée de n-heptane/isopropanol/diéthylamine dans les proportions 1000/25/1. Le premier composé élué correspond au produit titre avec un excès énantiomérique de 99 %, qui est transformé en chlorhydrate par action de l'éther chlorhydrique.
*Point de fusion (M.K.): 228-232°C*

| $[\alpha]^{25°C}$ (c = 1 % dans $CH_3OH$) | |
|---|---|
| λ(nm) | α |
| 589 | + 16,5 |
| 578 | + 17,2 |
| 546 | + 19,7 |
| 436 | + 35,9 |
| 365 | + 61,5 |

**EXEMPLE 8**

**Isomère (-) du 1-(N,N-diméthylaminométhyl)-1-(1-hydroxycyclohex-1-yl)-5-méthoxy benzocyclobutane et son chlorhydrate**

Le deuxième composé séparé au cours de la chromatographie mise en oeuvre à l'exemple 7 correspond au produit titre avec un excès énantiomérique de 99 %, qui est transformé en son chlorhydrate.
*Point de fusion (M.K.) : 228-232°C*

| $[\alpha]^{25°C}$ (c = 1 % dans $CH_3OH$) | |
|---|---|
| λ(nm) | α |
| 589 | - 16,6 |
| 578 | - 17,3 |
| 546 | - 20,0 |
| 436 | - 36,7 |
| 365 | - 63,3 |

**EXEMPLE 9**

**1-(N,N-diméthylaminométhyl)-1-(1-hydroxycyclohex-1-yl)-6-méthoxybenzocyclobutane**

*Stade G : 6-méthoxybenzocyclobutane-1-ol*

Sur une solution de 42,8 g (0,29 mole) de 6-méthoxybenzocyclobutane-1-one dans 1,5 l de méthanol maintenue à 0°C, 13,21 g (0,35 mole) de borohydrure de sodium sont additionnés par petites fractions. La température est maintenue à 0° C pendant 2 heures. Après retour à température ambiante, le milieu réactionnel est évaporé à sec puis repris à l'eau. La phase aqueuse est extraite plusieurs fois à l'acétate d'éthyle. Les phases organiques sont réunies, lavées par de la saumure, séchées sur $MgSO_4$, filtrées et évaporées pour donner après chromatographie sur silice 29,2 g de produit attendu sous forme d'un solide dont le point de fusion est 71-73° C.

*Stade H : 1-bromo 6-méthoxybenzocyclobutane*

Sur une solution de 29,1 g (0,194 mole) de produit obtenu au stade précédent dans 940 ml de $NaHCO_3$ à 0°C, on coule lentement 13,5 ml (0,144 mole) de tribromure de phosphore. Le mélange est conservé à cette température pendant 20 minutes, puis hydrolysé par 900 ml de $NaHCO_3$ à 0°C. La phase aqueuse est extraite plusieurs fois à l'éther. Les phases éthérées réunies sont lavées à l'eau, séchées sur $MgSO_4$, filtrées et évaporées pour donner 23,1 g d'une huile jaune qui correspond à la structure du produit attendu.

*Stade I : 1-cyano 6-méthoxybenzocyclobutane*

A une solution de 22,9 g (0,1 mole) de produit obtenu au stade précédent dans 240 ml de diméthylsulfoxyde, sont additionnés rapidement 10,5g (0,16 mole) de cyanure de potassium. Ce mélange est alors chauffé à 55° C pendant 4 heures. Après retour à température ambiante, le milieu réactionnel est versé sur deux litres d'eau. La phase aqueuse est extraite plusieurs fois à l'éther. Les phases organiques sont lavées à l'eau, séchées sur $MgSO_4$, filtrées et évaporées. Le résidu est chromatographié sur silice (éluant $CH_2Cl_2$/cyclohexane : 50/50) pour donner 10,4 g de produit attendu qui fond à 58-59°C.

*Stade J : 1-cyano 1-(1-hydroxycyclohex-1-yl) 6-méthoxybenzocyclobutane*

On procède comme dans le stade A de l'exemple 1 en utilisant comme substrat le produit obtenu au stade I.
*Point de fusion (K.) : 116-120°C*

*Stade K : 1-aminométhyl 1-(1-hydroxycyclohex-1-yl) 6-méthoxybenzocyclobutane*

On procède comme dans le stade B de l'exemple len utilisant comme substrat le produit obtenu au stade J.
*Point de fusion (K.): 90-92° C*

*Stade L : 1-(N,N-diméthylaminométhyl)-1-(1-hydroxycyclohex-1-yl)-6-méthoxybenzocyclobutane*

On procède comme dans le stade C de l'exemple 1, à partir du composé obtenu dans le stade K.
*Point de fusion (M.K): 125-133°C*

**EXEMPLE 10**

**1-(N,N-diméthylaminométhyl)-1-(4-hydroxy tétrahydro-4H-pyran-4-yl)-4-méthoxybenzocyclobutane**

On procède comme dans l'exemple 1 en utilisant respectivement au stade A comme substrats le 1-cyano 4-méthoxybenzocyclobutane et la tétrahydro-4*H*-pyran-4-one.
*Point de fusion (K.): 88-90°C (base libre)*

**EXEMPLE 11**

**Chlorhydrate de (+) 1-(N,N-diméthylaminométhyl)-1-(1-hydroxycyclohex-1-yl)-4-méthoxybenzocyclobutane**

2 g du composé de l'exemple 4 sont séparés sur colonne chirale de type CHIRALCEL OD® par H.P.L.C., la

phase mobile étant composé de n-heptane/isopropanol/$CF_3CO_2H$ dans les proportions 1000/30/1. Le premier composé élué correspond au produit attendu avec un excès énantiomérique de 99,5 %, qui est transformé en chlorhydrate par action de l'éther chlorhydrique.
*Point de fusion (M.K.) : 194-206°C*

| $[\alpha]^{25°C}$ (c = 1 % dans $CH_3OH$) | |
|---|---|
| λ(nm) | α |
| 578 | + 0,98 |
| 546 | + 1,33 |
| 436 | + 4,78 |

**EXEMPLE 12**

**Chlorhydrate de (-) 1-(N,N-diméthylaminométhyl)-1-(1-hydroxycyclohex-1-yl)-4-méthoxybenzocyclobutane**

Le deuxième composé séparé au cours de la chromatographie mise en oeuvre à l'exemple 11 correspond au produit attendu avec un excès énantiomérique de 99 %. Il est transformé en chlorhydrate par l'éther chlorhydrique.
*Point de fusion (M.K.): 210-212°C*

| $[\alpha]^{25°C}$ (c = 1 % dans $CH_3OH$) | |
|---|---|
| λ(nm) | α |
| 578 | - 1,32 |
| 546 | - 1,6 |
| 436 | - 5,94 |

**EXEMPLE 13**

**Chlorhydrate de 1-(N,N-diméthylaminométhyl)-1-(1-hydroxycyclohex-1-yl)-4-méthoxy-5-chlorobenzocyclobutane**

On procède comme dans l'exemple 1 en utilisant comme substrat au stade A le 5-chloro-1-cyano-4-méthoxybenzocyclobutane.
*Point de fusion (M.K.) : 230-235°C*

**EXEMPLE 14**

**Chlorhydrate de 1-(N,N-diméthylaminométhyl)-1-(1-hydroxycyclohex-1-yl)-3-fluorobenzocyclobutane**

On procède comme dans l'exemple 1 en utilisant comme substrat au stade A le 1-cyano-3-fluorobenzocyclobutane.
*Point de fusion (M.K.) : 240-245°C*

**EXEMPLE 15**

**Chlorhydrate de 1-(N,N-diméthylaminométhyl)-1-(1-hydroxycyclohex-1-yl)-3-chlorobenzocyclobutane**

On procède comme dans l'exemple 1 en utilisant comme substrat au stade A le 3-chloro-1-cyanobenzocyclobutane.
*Point de fusion (M.K.) : 242-245°C*

**EXEMPLE 16**

**1-(N,N-diméthylaminométhyl)-1-(4-hydroxytétrahydro-4*H*-thiopyran-4-yl)-5-méthoxybenzocyclobutane**

On procède comme dans l'exemple 1 en utilisant respectivement comme substrat le 1-cyano-5-méthoxybenzocyclobutane et le tétrahydro-4*H*-thiopyran-4-one.
*Point de fusion (M.K.) : 118-123°C*

**EXEMPLE 17**

**Chlorhydrate de 1-(N,N-diméthylaminométhyl)-1-(1-hydroxycyclohex-1-yl)-4-trifluorométhylbenzocyclobutane**

On procède comme dans l'exemple 1 en utilisant comme substrat au stade A le 1-cyano-4-trifluorométhylbenzocyclobutane.
*Point de fusion (M.K.) : 208-212°C*

**EXEMPLE 18**

**Chlorhydrate de 1-(N,N-diméthylaminométhyl)-1-(1-hydroxycyclohex-1-yl)-5-fluorobenzocyclobutane**

On procède comme dans l'exemple 1 en utilisant comme substrat au stade A le 1-cyano-5-fluorobenzocyclobutane.
*Point de fusion (M.K.): 233-238°C*

**EXEMPLE 19**

**1-Aminométhyl-1-(1-hydroxycyclohex-1-yl)-4-hydroxybenzocyclobutane**

On procède comme dans l'exemple 1, des stades A à B, en utilisant comme substrat au stade A le 1-cyano-4-hydroxybenzocyclobutane.
*Point de fusion (M.K.): 185-190°C*

**EXEMPLE 20**

**1-(N,N-diméthylaminométhyl)-1-(1-hydroxycyclohex-1-yl)-4-hydroxybenzocyclobutane**

Le composé obtenu dans l'exemple 19, traité selon les conditions opératoires décrites dans le stade C de l'exemple 1, permet d'obtenir le produit attendu.
*Point de fusion (M.K.) : 222-225°C*

**EXEMPLE 21**

**1-Aminométhyl-1-(1-hydroxycyclohex-1-yl)-5-hydroxybenzocyclobutane**

On procède comme dans l'exemple 19 en utilisant comme substrat de départ le 1-cyano-5-hydroxybenzocyclobutane.
*Point de fusion (M.K.): 185-190°C*

**EXEMPLE 22**

**1-(N,N-diméthylaminométhyl)-1-(1-hydroxycyclohex-1-yl)-5-hydroxybenzocyclobutane**

Le composé obtenu dans l'exemple 21, traité selon les conditions opératoires décrites dans le stade C de l'exemple 1, permet d'obtenir le produit attendu.
*Point de fusion (M.K.) : 177-182°C*

**EXEMPLE 23**

**1-(N,N-diméthylaminométhyl)-1-(1-hydroxycyclohex-1-yl)-4,5-dichlorobenzocyclobutane**

On procède comme dans l'exemple 1 en utilisant comme substrat au stade A le 4,5-dichloro-1-cyanobenzocyclobutane.
*Point de fusion (M.K.) : 135-137°C*

**EXEMPLE 24**

**Chlorhydrate de 1-(N,N-diméthylaminométhyl)-1-(1-hydroxycyclohex-1-yl)-6-chlorobenzocyclobutane**

On procède comme dans l'exemple 1 en utilisant comme substrat au stade A le 1-cyano-6-chlorobenzoycyclobutane.
*Point de fusion (M.K.) : 195-205°C*

**EXEMPLE 25**

**Chlorhydrate de 1-(N,N-diméthylaminométhyl)-1-(4-*tert*-butyl-1-hydroxycyclohex-1-yl)-5-méthoxybenzocyclobutane**

On procède comme dans l'exemple 1 en utilisant respectivement comme substrats au stade A le 1-cyano-5-méthoxybenzocyclobutane et la 4-tert-butyl-cyclohexanone.
*Point de fusion (M.K.): 206-220°C*

**EXEMPLE 26**

**Chlorhydrate de 1-(N,N-diméthylaminométhyl)-1-(4,4-diméthyl-1-hydroxycyclohex-1-yl)-5-méthoxybenzocyclobutane**

On procède comme dans l'exemple 1 en utilisant respectivement comme substrat au stade A le 1-cyano-5-méthoxybenzocyclobutane et la 4,4-diméthylcyclohexanone.
*Point de fusion (M.K.) : 180-215°C (sublimation entre 210 et 212°C)*

**EXEMPLE 27**

**1-(N,N-diméthylaminométhyl)-1-(1-hydroxy-4-sélénocyclohex-1-yl)-5-méthoxybenzocyclobutane**

On procède comme dans l'exemple 1 en utilisant respectivement comme substrat au stade A le 1-cyano-5-méthoxybenzocyclobutane et la 4-sélénocyclohexanone.
*Point de fusion (M.K.) : 113-120°C*

**EXEMPLE 28**

**1-(N,N-diméthylaminométhyl)-1-(4-hydroxy-1-méthyl-pipéridin-4-yl)-5-méthoxybenzocyclobutane**

On procède comme dans l'exemple 1 en utilisant respectivement comme substrat au stade A le 1-cyano-5-méthoxybenzocyclobutane et la 1-méthyl-4-pipéridinone.
*Point de fusion (M.K.): 113-116°C*

**EXEMPLE 29**

**Fumarate de 1-(N,N-diméthylaminométhyl)-1-(1-hydroxycyclohex-1-yl)-5-(trifluorométhylsulfonate)benzocyclobutane**

On procède comme dans l'exemple 1 en utilisant comme substrat au stade A le 1-cyano-5-(trifluorométhylsulfonate)benzocyclobutane.
*Point de fusion (M.K.) : 72-77°C*

**EXEMPLE 30**

**1-(N,N-diméthylaminométhyl)-1-(1-hydroxycyclohex-1-yl)-6-bromobenzocyclobutane**

On procède comme dans l'exemple 1 en utilisant comme substrat au stade A le 1-cyano-6-bromobenzocyclobutane.
*Point de fusion (M.K.) : 196-200°C*

**EXEMPLE 31**

**1(N-imidazolin-2-yl-aminométhyl)-1-(1-hydroxycyclohex-1-yl)4-méthoxybenzocyclobutane**

2 g du composé de l'exemple 5 sont dissous dans une solution contenant 1,16 ml de triéthylamine, 1,2 g d'acide imidazolin-2-yl sulfonique et 16 ml d'acétonitrile et portés à reflux pendant 3 heures. On dilue au chlorure de méthylène, lave à la soude normale puis à l'eau, sèche sur $MgSO_4$ et évapore. On cristallise de l'éther pour obtenir 600 mg d'un solide qui correspond au produit attendu.

**EXEMPLE 32**

**1-[2-(N,N-diméthylamino)éthyl]-1-(1-hydroxycyclohex-1-yl)-5-méthoxybenzocyclobutane**

*Stade 1 : 1-[2-(N,N-diméthylamino)éthyl]-1-cyano-5-méthoxybenzocyclobutane*

On refroidit à -78°C une solution de 10,5 ml de diisopropylamine dans 55 ml de tétrahydrofurane, puis on ajoute goutte à goutte 47 ml d'une solution 1,6 M de n-butyllithium dans le cyclohexane. On laisse agiter 15 minutes à -78°C puis on ajoute rapidement 10,8 g de chlorhydrate de 2-chloroéthyldiméthylamine. On laisse agiter 30 minutes à -78°C.
D'autre part, on prépare dans un second tricol une solution refroidie à -25°C de 8,8 ml de diisopropylamine dans 75 ml de tétrahdyrofurane. On ajoute à cette solution 39,2 ml d'une solution 1,6 M de n-butyllithium dans le cyclohexane. Après 15 minutes à -25°C, on ajoute à ce milieu réactionnel une solution de 10 g de 1-cyano-5-méthoxybenzocyclobutane dans 75 ml de tétrahydrofurane. On laisse agiter 30 minutes à -25°C.

A l'aide d'une canule, on transvase la première solution dans le seconde à -78°C. On laisse agiter à -78°C pendant 1 heure puis on abandonne la nuit à température ambiante. On hydrolyse à 0°C par du chlorure d'ammonium. On extrait à l'éther, sèche la phase organique sur $MgSO_4$, filtre et évapore à sec. Le résidu obtenu est repris par 250 ml d'HCl (1N), lavé à l'éther, puis la phase aqueuse est amenée à pH = 12 par de la soude à 20 %. On extrait au chlorure de méthylène, lave à l'eau, sèche au $MgSO_4$ et évapore. On obtient 12,9 g du produit attendu sous forme d'une huile jaune pâle.

*Stade 2 : 1-[2-(N,N-diméthylamino)éthyl]-1-méthoxycarbonyl-5-méthoxybenzocyclobutane*

6 g du produit obtenu au stade précédent sont solubilisés dans 100 ml de méthanol et 100 ml de chlorure de méthylène. La solution est refroidie à 0°C et on fait buller de l'acide chlorhydrique gazeux pendant 15 minutes. On abandonne à température ambiante pendant 3 jours. Après concentration sous pression réduite, le résidu est solubilisé dans l'eau et neutralisé à froid par une solution de bicarbonate de sodium à 10 % dans l'eau. Une chromatographie sur gel de silice (dichlorométhane/méthanol : 90/10) permet d'isoler le produit attendu.

*Stade 3 : 1-[2-(N,N-diméthylamino)éthyl]-1-(1-hydroxycyclohex-1-yl)-5-méthoxybenzocyclobutane*

5 g du produit obtenu au stade précédent en solution dans 50 ml de tétrahydrofurane sont additionnés lentement sur 45,2 ml d'une solution 0,5 M de pentane 1,5-(dimagnésium bromure) dans le tétrahydrofurane en maintenant la température à 0°C. On laisse agiter 15 minutes à 0°C puis 1 heure à température ambiante. On verse sur une solution aqueuse saturée en chlorure d'ammonium. On extrait à l'éther, sèche sur sulfate de magnésium, filtre et évapore à sec. Une chromatographie sur gel de silice (dichlorométhane/éthanol : 90/10) permet d'isoler le produit attendu.

**ETUDE PHARMACOLOGIQUE DES COMPOSES DE L'INVENTION**

*A. Etude in vitro*

**EXEMPLE 33**

**Détermination de l'affinité pour les sites de recapture de la sérotonine**

L'affinité a été déterminée par des expériences de compétition avec la [$^3$H]-paroxetine (NEN, Les Ulis, France). Les membranes sont préparées à partir de cortex frontal de rat et incubées en triple avec 1,0 nM de [$^3$H]-paroxetine et le ligand froid dans un volume final de 0,4 ml, pendant 2 heures à 25° C. Le tampon d'incubation contient 50 nM de TRIS-HCl (pH 7,4), 120 mM de NaCl et 5 mM de KCl. La fixation non-spécifique est déterminée avec 10 µM de citalopram. A la fin de l'incubation, le milieu d'incubation est filtré et lavé trois fois avec 5 ml de tampon refroidi. La radioactivité retenue sur les filtres est déterminée par comptage du liquide de scintillation. Les isothermes de binding sont analysées par régression non-linéaire pour déterminer les valeurs d'$IC_{50}$. Elles sont converties en constante de dissociation ($K_i$) par l'intermédiaire de l'équation de Cheng-Prusoff :

$$K_i=IC_{50}/(1+L/K_d)$$

dans laquelle L est la concentration de [$^3$H]-paroxetine et $K_d$ est la constante de dissociation de [$^3$H]-paroxetine pour le site de recapture de la sérotonine (0,13 nM). Les résultats sont exprimés en $pK_i$ (- log $K_i$).

Les composés de la présente invention montrent une très bonne affinité pour les sites de recapture de la sérotonine. A titre d'exemple le $pK_i$ du composé de l'exemple 8 est de 8,7. Par comparaison le $pK_i$ de la fluoxétine dans ce test est de 8.

**EXEMPLE 34**

**<u>Détermination de l'affinité pour les sites de recapture de la noradrénaline</u>**

L'affinité a été déterminée par des expériences de compétition avec la [$^3$H]-nisoxétine (Amersham, les Ulis, France). Les membranes sont préparées à partir de cortex frontal de rat et incubées en triple avec 2 nM de [$^3$H]-nisoxétine et le ligand froid dans un volume final de 0,5 ml, pendant 4 heures à 4° C. Le tampon d'incubation contient 50 mM de TRIS-HCl (pH 7,4), 300 mM de NaCl et 5 mM de KCl. La fixation non-spécifique est déterminée avec 10 µM de desipramine. A la fin de l'incubation, le milieu d'incubation est filtré et lavé trois fois avec 5 ml de tampon de filtration refroidi (50 mM de TRIS-HCl, pH 7,4, 300 mM de NaCl et 5 mM de KCl). La radioactivité retenue sur les filtres est déterminée par comptage du liquide de scintillation. Les isothermes de binding sont analysées par régression non-linéaire pour déterminer les valeurs d'$IC_{50}$. Elles sont converties en constante de dissociation ($K_i$) par l'intermédiaire de l'équation de Cheng-Prusoff:

$$K_i=IC_{50}/(1+L/K_d)$$

dans laquelle L est la concentration de [$^3$H]-nisoxetine et $K_d$ est la constante de dissociation de [$^3$H]-nisoxetine pour le site de recapture de la noradrénaline (1,23 nM). Les résultats sont exprimés en pKi (-log Ki).

A titre d'exemple, le composé de l'exemple 9 a un pKi de 7,25.

***B. <u>Etude in vivo</u>***

**EXEMPLE 35**

**<u>Test de la nage forcée chez la souris (Porsolt et al., 1977)</u>**

Le test de la nage forcée chez la souris consiste à induire chez l'animal un "état de désespoir" en le plaçant pendant 6 minutes dans un cylindre rempli d'eau d'où il ne peut s'échapper. L'animal naïf se débat vigoureusement pendant les premières minutes, puis adopte une posture immobile pendant les dernières minutes de l'essai. Les produits antidépresseurs réduisent le temps d'immobilité de l'animal lors du test.

Les animaux sont des souris mâles CD (IFFA-CREDO) (22-26 g) placées individuellement la veille du test dans des cages plastiques transparentes (25x15x14 cm) sur sciure, avec nourriture et boisson à volonté. Le jour du test, 30 minutes après le traitement (produit ou solvant, administré par voie sous-cutanée), chaque souris est plongée pendant 6 minutes (T0-T6) dans un cylindre en verre (23,5 cm H., 11,5 cm diam.) rempli d'eau à 24 ± 0.5° C, jusqu'à une hauteur de 6 centimètres. On note la durée totale d'immobilité (sec) de l'animal pendant les 4 dernières minutes, du test (T2-T6): une souris est considérée immobile quand elle flotte dans l'eau, en faisant seulement des petits mouvements pour maintenir sa tête hors de l'eau.

Les différences entre lots traités (produit) et lot témoin (solvant) sont évaluées statistiquement par analyse de variance suivie du test de Dunnett's, avec $P < 0,05$.

A titre d'exemple et pour illustrer les effets des produits de l'invention on rapporte dans le tableau suivant les résultats du composé de l'exemple 4.

| Traitement | Doses mg/kg s.c. | Durée immobilité (sec) | %/t témoin | N |
|---|---|---|---|---|
| Témoin solvant | | 167,1 ± 16,1 | | 6 |
| Produit de l'exemple 4 | 0,63 | 157,3 ± 18,9 | 94 | 3 |
| | 2,5 | 101,6 ± 57,3 | 61 | 3 |
| | 10,0 | 6,1 ± 6,1 * | 4 | 4 |
| Témoin solvant | | 173,6 ± 10,1 | | 12 |
| fluoxétine | 2,5 | 163,5 ± 33,4 | 94 | 5 |
| | 10,0 | 130,5 ± 23,5 | 75 | 7 |
| | 40,0 | 127,1 ± 25,2 | 73 | 8 |

* P<0,05

## EXEMPLE 36

**Test de suspension par la queue chez la souris**

Dans cette variante du test de nage forcée, on induit l'état de "désespoir" chez la souris en suspendant celle-ci par la queue, la tête en bas. Placé dans cette situation inconfortable, l'animal se débat vigoureusement au début, puis adopte une posture immobile. Les produits antidépresseurs réduisent le temps d'immobilité de l'animal.
Les animaux sont des souris mâles NMRI (IFFA-CREDO) (22-26 g) stabulées par groupe de 20, dans des cages plastiques transparentes (59x38x20 cm) sur sciure, avec nourriture et boisson à volonté. Le jour du test, chaque souris est placée en cage individuelle dès l'administration (sous-cutanée) du produit ou solvant. Trente minutes après, l'animal est suspendu par la queue à un crochet, au moyen d'un ruban adhésif fixé sur la queue. Le crochet est relié à un capteur de tension qui transmet tous les mouvements de l'animal à un processeur central (système ITEMATIC-TST 1, ITEM-LABO, France). Le temps total (sec) d'immobilité de l'animal pendant les 6 minutes de test est automatiquement enregistré.
Les différences de temps d'immobilité entre lots traités (produit) et lot témoin (solvant) sont analysées statistiquement par ANOVA suivie du test de Dunnett's, avec P < 0,05.
A titre d'exemple et pour illustrer les effets des produits de l'invention on rapporte dans le tableau ci-dessous les résultats du composé de l'exemple 3.

| Traitement | Doses mg/kg s.c. | Immobilité (sec) | %/t témoin | N |
|---|---|---|---|---|
| Témoin solvant | 0 | 80,3 ± 14,3 | | 12 |
| Produit de l'exemple 3 | 0,63 | 70,4 ± 13,5 | 88 | 8 |
| | 2,5 | 41,1 + 9,6 | 51 | 8 |
| | 10,0 | 27,8 ± 9,6 * | 35 | 8 |
| Témoin solvant | | 76,6 ± 11,3 | | 17 |
| fluoxétine | 40,0 | 69,9 ± 17,8 | 91 | 10 |
| | 80,0 | 84,3 ± 12,4 | 110 | 10 |

* P<0,05.

Les résultats sur les tests ci-dessus, illustrent l'excellente activité des produits de l'invention, notamment en comparaison avec le produit de référence.

**Revendications**

**1.** Composés de formule (I) :

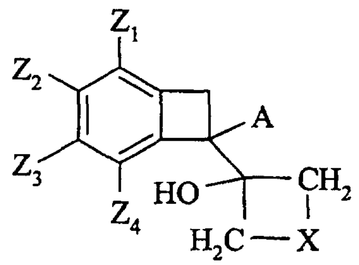

(I)

dans laquelle :

$Z_1$, $Z_2$, $Z_3$ et $Z_4$, identiques ou différents, représentent chacun un atome d'hydrogène, d'halogène, un groupement $(C_1\text{-}C_6)$alkyle linéaire ou ramifié, $(C_2\text{-}C_6)$alkényle linéaire ou ramifié, $(C_2\text{-}C_6)$alkynyle linéaire ou ramifié, $[(C_3\text{-}C_8)$cycloalkyl$]\text{-}(C_1\text{-}C_6)$ alkyle, la partie alkyle étant linéaire ou ramifié, hydroxy, $(C_1\text{-}C_6)$alkoxy linéaire ou ramifié, benzyloxy
$(C_2\text{-}C_6)$alkénoxy linéaire ou ramifié, $(C_2\text{-}C_6)$alkynoxy linéaire ou ramifié, cyano, trifluorométhyle, trifluorométhoxy, nitro, un groupement de formule $-OSO_2CF_3$, $OSO_2CH_3$, $-NHCOCH_3$, $-NHCOCF_3$, $-NHSO_2CH_3$,

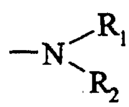

ou un groupement de formule

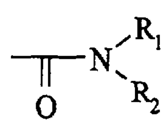

dans lesquelles
$R_1$ et $R_2$, identiques ou différents, représentent chacun un atome d'hydrogène, un groupement $(C_1\text{-}C_6)$alkyle linéaire ou ramifié, $(C_2\text{-}C_6)$alkényle linéaire ou ramifié, $(C_2\text{-}C_6)$alkynyle linéaire ou ramifié, arylalkyl $(C_1\text{-}C_6)$ linéaire ou ramifié ou $[(C_3\text{-}C_8)$cycloalkyl$]\text{-}(C_1\text{-}C_6)$alkyle, la partie alkyle étant linéaire ou ramifié,
X représente :

- un atome d'oxygène,
- un groupement de formule $S(O)_p$ dans lequel p représente un entier compris entre 0 et 2 inclus,
- un groupement de formule $-(CH_2)_n$ dans lequel n représente un entier compris entre 1 et 4 inclus,
- ou un groupement de formule $-CH_2\text{-}Y\text{-}CH_2\text{-}$ dans lequel Y représente un atome d'oxygène, un atome de sélénium, un groupement $\rangle S(O)_p$ avec p tel que défini précédemment, $\rangle N\text{-}R_1$ ou

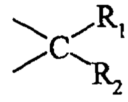

dans lesquels $R_1$ et $R_2$ ont la même signification que précédemment,

A représente un groupement de formule :

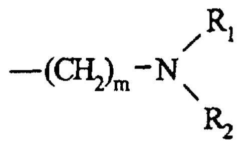

ou

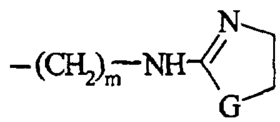

dans lesquels
$R_1$ et $R_2$ ont la signification précédemment définie,
m représente un nombre entier compris entre 1 et 6 inclus, et
G représente un atome d'oxygène ou le groupe NH,

leurs isomères ainsi que leurs sels d'addition à un acide pharmaceutiquement acceptable.

**2.** Composés de formule (I) selon la revendication 1 **caractérisés en ce que** A représente un groupement de formule

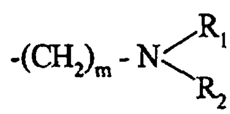

dans laquelle m, $R_1$ et $R_2$ sont tels que définis dans la formule (I), leurs isomères ainsi que leurs sels d'addition à une acide pharmaceutiquement acceptable.

**3.** Composés de formule (I) selon la revendication 1 **caractérisés en ce que** X représente un groupement de formule $-(CH_2)_n-$ dans laquelle n est tel que défini dans la formule (I), leurs isomères ainsi que leurs sels d'addition à un acide pharmaceutiquement acceptable.

**4.** Composés de formule (I) selon l'une quelconque des revendications 1 à 3, **caractérisés en ce que** :

- A représente un groupement de formule

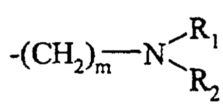

avec $R_1$ et $R_2$ tels que définis précédemment et m est égal à 1,
- et X représente un groupement de formule $-(CH_2)_n-$ avec n est égal à 3,

leurs isomères ainsi que leurs sels d'addition à un acide pharmaceutiquement acceptable.

**5.** Composés de formule (I) selon la revendication 1 **caractérisés en ce que** X représente un groupement de formule $-CH_2-Y-CH_2-$ dans laquelle Y est tel que défini dans la formule (I), leurs isomères ainsi que leurs sels d'addition à un acide pharmaceutiquement acceptable.

6. Composé de formule (I) selon l'une quelconque des revendications 1 à 4 qui est le 1-(N,N-diméthylaminométhyl)-1-(1-hydroxycyclohex-1-yl)-5-méthoxybenzocyclobutane, et ses sels d'addition à un acide pharmaceutiquement acceptable.

7. Composé de formule (I) selon l'une quelconque des revendications 1 à 4 qui est le 1-(N,N-diméthylaminométhyl)-1-(1-hydroxycyclohex-1-yl)-4-méthoxybenzocyclobutane, et ses sels d'addition à un acide pharmaceutiquement acceptable.

8. Composé de formule (I) selon l'une quelconque des revendications 1 à 4 qui est le (-)-1-(N,N-diméthylaminométhyl)-1-(1-hydroxycyclohex-1-yl)-5-méthoxybenzocyclobutane, et ses sels d'additions à un acide pharmaceutiquement acceptable.

9. Procédé de préparation des composés de formule (I) selon la revendication 1 **caractérisé en ce que** l'on utilise comme produit de départ :

*un composé de formule (II)* :

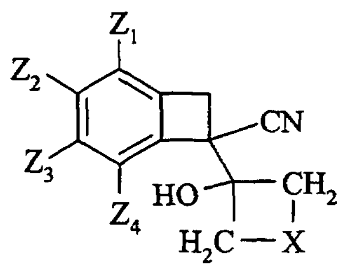

(II)

dans laquelle $Z_1$, $Z_2$, $Z_3$, $Z_4$ et X sont tels que définis dans la formule (I),
que l'on traite par un hydrure dans l'éther ou le tétrahydrofurane, pour conduire aux composés de formule (I/a), cas particulier des composés de formule (I):

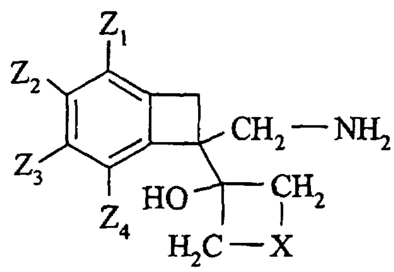

(I/a)

dans laquelle $Z_1$, $Z_2$, $Z_3$, $Z_4$ et X ont les significations précédemment définies,
composés de formule (I/a) que l'on substitue en utilisant les méthodes classiques de la chimie organique telles que :

- amination réductrice à partir des aldéhydes ou cétones correspondants, en présence de cyanoborohydrure de sodium ou de triacétoxyborohydrure de sodium, ou
- substitution nucléophile de dérivés de formule (III):

$$R'_1Z \qquad (III)$$

dans laquelle $R'_1$ prend la signification précédemment définie pour $R_1$ à l'exception de la valeur hydrogène, et Z est un groupe partant tel que, I, Br, Cl,

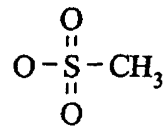

ou

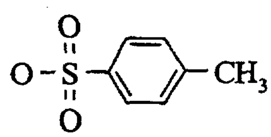

pour conduire aux amines secondaires de formule (I/b), cas particulier des composés de formule (I):

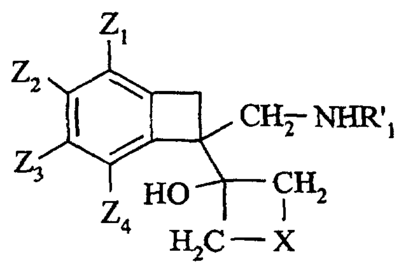

(I/b)

dans laquelle $Z_1$, $Z_2$, $Z_3$, $Z_4$, X et $R'_1$ ont les significations précédemment définies,
composés de formule (I/b) sont à nouveau substituées par les mêmes méthodes que celles décrites ci-dessus pour conduire aux amines tertiaires de formule (Vc), cas particulier des composés (I):

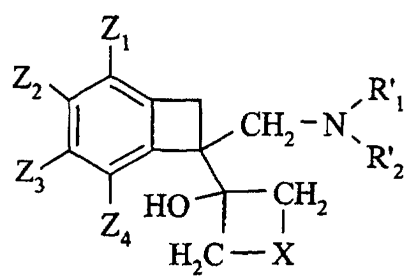

(I/c)

dans laquelle $Z_1$, $Z_2$, $Z_3$, $Z_4$ et X prennent les valeurs précédemment définies et $R'_1$ et $R'_2$ ont respectivement les significations précédemment définies pour $R_1$ et $R_2$ à l'exception de la valeur hydrogène,
étant entendu que lorsque $R'_1$ et $R'_2$ sont identiques tout en étant chacun différent d'hydrogène, les amines tertiaires de formule (I/c) sont obtenues directement à partir de l'amine (I/a) sans avoir à isoler l'amine secondaire (I/b).

**10.** Procédé de préparation des composés de formule (I) selon la revendication 1 **caractérisé en ce que** l'on utilise comme produit de départ :

<u>*un composé de formule (IV)*</u> :

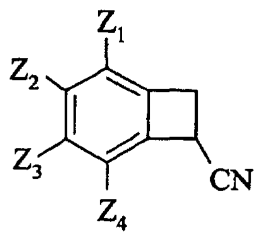

(IV)

dans laquelle $Z_1$, $Z_2$, $Z_3$, $Z_4$ sont tels que définis dans la formule (I), que l'on substitue, en présence d'une base forte, par une amine de formule (V) :

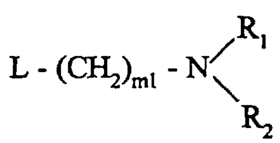

(V)

dans laquelle $R_1$ et $R_2$ sont tels que définis dans la formule (I), ml est un entier compris entre 2 et 6 inclus et L représente un groupe partant tel qu'un atome d'halogène, un groupement mésylate, tosylate, ou trifluorométhanesulfonate,
pour conduire aux composés de formule (VI):

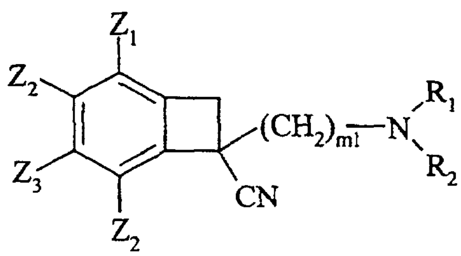

(VI)

dans laquelle $Z_1$, $Z_2$, $Z_3$, $Z_4$, $R_1$, $R_2$ et $m_1$ sont tels que définis précédemment,
composés de formule (VI) que l'on traite dans des conditions d'hydrolyse alcoolique acide en présence d'un composé de formule (VII):

$$G - OH \qquad (VII)$$

dans laquelle G représente un groupement alkyle ($C_1$-$C_6$) linéaire ou ramifié,
pour conduire aux composés de formule (VIII):

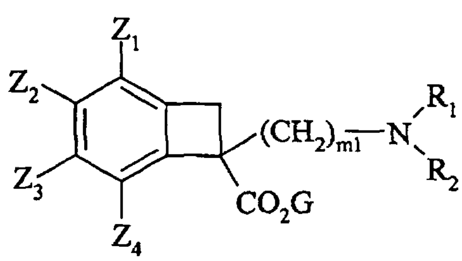

(VIII)

dans laquelle $Z_1$, $Z_2$, $Z_3$, $Z_4$, $R_1$, $R_2$, G et m1 sont tels que définis précédemment,
composés de formule (VIII) que l'on traite par un dimagnésien de formule (IX) :

$$Z'\text{-}Mg\text{-}CH_2\text{-}X\text{-}CH_2\text{-}Mg\text{-}Z' \qquad (IX)$$

dans laquelle X est tel que défini dans la formule (I) et Z' représente un atome d'halogène, pour conduire aux composés de formule (I/d), cas particulier des composés de formule (I):

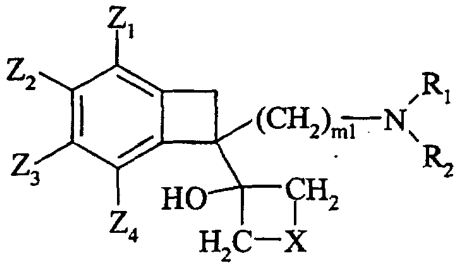
(I/d)

dans laquelle $Z_1$, $Z_2$, $Z_3$, $Z_4$, $R_1$, $R_2$, X et m1 sont tels que définis précédemment.

**11.** Procédé selon l'une des revendications 9 et 10 **caractérisé en ce que** les composés de formule (I/e):

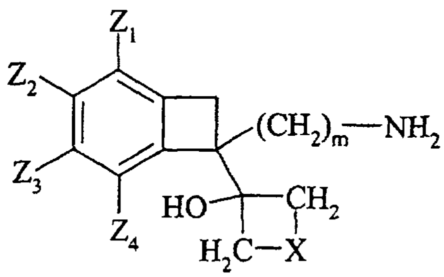
(I/e)

dans laquelle $Z_1$, $Z_2$, $Z_3$, $Z_4$, X et m sont tels que définis dans la formule (I), sont *traités par l'isocyanate de 2-chloroéthyle* dans l'acétonitrile en présence de triéthylamine pour conduire aux composés de formule (I/f), cas particulier des composés de formule (I):

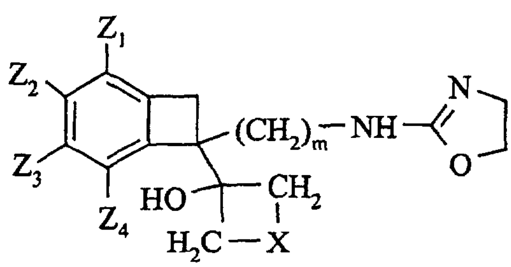
(I/f)

dans laquelle $Z_1$, $Z_2$, $Z_3$, $Z_4$, X et m ont les significations précédemment définies,

**12.** Procédé selon l'une des revendications 9 et 10 **caractérisé en ce que** les composés de formule (I/e) :

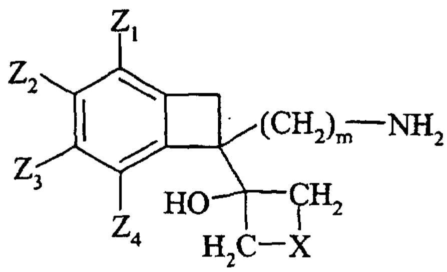

(I/e)

dans laquelle $Z_1$, $Z_2$, $Z_3$, $Z_4$, X et m sont tels que définis dans la formule (I), sont *traités par l'acide imidazolin-2-yl sulfonique* en présence d'une base organique ou minérale dans un alcool ou l'acétonitrile pour conduire aux composés de formule (I/g):

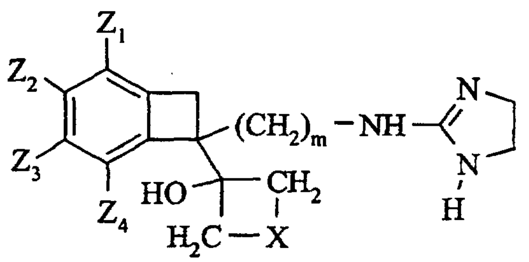

(I/g)

dans laquelle $Z_1$, $Z_2$, $Z_3$, $Z_4$, X et m ont les significations précédemment définies,

**13.** Compositions pharmaceutiques contenant comme principe actif au moins un composé de formule (I) selon l'une quelconque des revendications 1 à 8, seul ou en combinaison avec un ou plusieurs excipients ou véhicules inertes, non toxiques, pharmaceutiquement acceptables.

**14.** Compositions pharmaceutiques selon la revendication 10 contenant au moins un principe actif selon l'une quelconque des revendications 1 à 8 utiles dans le traitement de la dépression, des attaques de panique, des troubles obsessionnels compulsifs, des phobies, des troubles impulsifs, de l'abus de drogue et de l'anxiété.

**Patentansprüche**

**1.** Verbindungen der Formel (I):

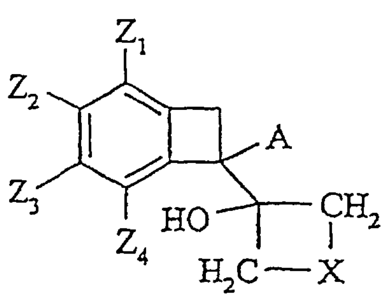

(I)

in der:

$Z_1$, $Z_2$, $Z_3$ und $Z_4$, die gleichartig oder verschieden sind, jeweils ein Wasserstoffatom, ein Halogenatom, eine geradkettige oder verzweigte ($C_1$-$C_6$)-Alkylgruppe, geradkettige oder verzweigte ($C_2$-$C_6$)-Alkenylgruppe, eine

geradkettige oder verzweigte ($C_2$-$C_6$)-Alkinylgruppe, (($C_3$-$C_8$)-Cycloalkyl]-($C_1$-$C_6$)-alkylgruppe, wobei der Alkylrest geradkettig oder verzweigte sein kann, Hydroxygruppe, geradkeltige oder verzweigte ($C_1$-$C_6$)-Alkoxygruppe, Benzyloxygruppe, geradkettige oder verzweigte ($C_2$-$C_6$)-Alkenoxygruppe, geradkettige oder verzweigte ($C_2$-$C_6$)-Alkinoxygruppe, Cyanogruppe, Trifluormethylgruppe, Trifluormethoxygruppe, Nitrogruppe, eine Gruppe der Formel $-OSO_2CF_3$, $OSO_2CH_3$, $-NHCOCH_3$, $-NHCOCF_3$, $-NHSO_2CH_3$,

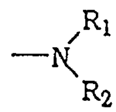

oder eine Gruppe der Formel

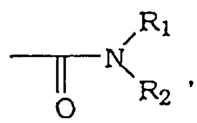

in denen

$R_1$ und $R_2$, die gleichartig oder verschieden sind, jeweils ein Wasserstoffatom, eine geradkettige oder verzweigte ($C_1$-$C_6$)-Alkylgruppe, geradkettige oder verzweigte ($C_2$-$C_6$)-Alkenylgruppe, geradkettige oder verzweigte ($C_2$-$C_6$)-Alkinylgruppe, geradkettige oder verzweigte Aryl-($C_1$-$C_6$)-alkylgruppe oder [($C_3$-$C_8$)-Cycloalkyl]-($C_1$-$C_6$)-alkylgruppe, wobei der Alkylrest geradkettig oder verzweigte sein kann, darstellen, bedeuten,

X:

- ein Sauerstoffatom,
- eine Gruppe der Formel $S(O)_p$, worin p eine ganze Zahl mit einem Wert zwischen 0 und 2 einschließlich darstellt,
- eine Gruppe der Formel $-(CH_2)_n$, worin n eine ganze Zahl zwischen 1 und 4 einschließlich darstellt.
- oder eine Gruppe der Formel $-CH_2-Y-CH_2-$, worin Y ein Sauerstoffatom, ein Seleniumatom, eine Gruppe $>S(O)_p$ , worin p die oben angegebenen Bedeutungen besitzt, $>N-R_1$ oder

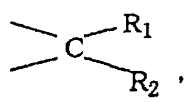

worin $R_1$ und $R_2$ die oben angegebenen Bedeutungen besitzen, darstellt, bedeutet,

A eine Gruppe der Formel:

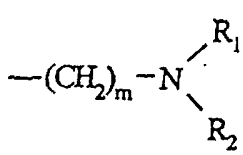

oder

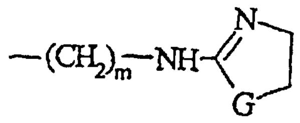 ,

worin
$R_1$ und $R_2$ die oben angegebenen Bedeutungen besitzen,
m eine ganze Zahl zwischen 1 und 6 einschließlich und
G ein Sauerstoffatom oder die Gruppe NH darstellen, bedeutet,

deren Isomere sowie deren Additionssalze mit einer pharmazeutisch annehmbaren Säure.

**2.** Verbindungen der Formel (I) nach Anspruch 1, **dadurch gekennzeichnet, daß** A eine Gruppe der Formel

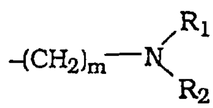

darstellt, worin m, $R_1$ und $R_2$ die bezüglich der Formel (I) angegebenen Bedeutungen besitzen, deren Isomere sowie deren Additionssalze mit einer pharmazeutisch annehmbaren Säure.

**3.** Verbindungen der Formel (I) nach Anspruch 1, **dadurch gekennzeichnet, daß** X eine Gruppe der Formel $-(CH_2)_n-$ bedeutet, worin n die bezüglich der Formel (I) angegebenen Bedeutungen besitzt, deren Isomere sowie deren Additionssalze mit einer pharmazeutisch annehmbaren Säure.

**4.** Verbindungen der Formel (I) nach einem der Ansprüche 1 bis 3, **dadurch gekennzeichnet, daß**:

- A eine Gruppe der Formel

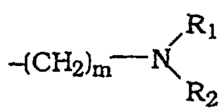

  darstellt, worin $R_1$ und $R_2$ die oben angegebenen Bedeutungen besitzen und m den Wert 1 besitzt,
- und X eine Gruppe der Formel $-(CH_2)_n-$ darstellt, worin n den Wert 3 besitzt. deren Isomere sowie der Additionssalze mit einer pharmazeutisch annehmbaren Säure.

**5.** Verbindungen der Formel (I) nach Anspruch 1, **dadurch gekennzeichnet, daß** X eine Gruppe der Formel $-CH_2-Y-CH_2-$ darstellt, worin Y die bezüglich der Formel (I) angegebenen Bedeutungen besitzt, deren Isomere sowie deren Additionssalze mit einer pharmazeutisch annehmbaren Säure.

**6.** Verbindung der Formel (I) nach einem der Ansprüche 1 bis 4, nämlich 1-(N,N-Dimethylaminomethyl)-1-(1-hydroxycyclohex-1-yl)-5-methoxybenzocyclobutan und dessen Additionssalze mit einer pharmazeutisch annehmbaren Säure.

**7.** Verbindung der Formel (I) nach einem der Ansprüche 1 bis 4, nämlich 1-(N,N-Dimethylaminomethyl)-1-(1-hydroxycyclohex-1-yl)-4-methoxybenzocyclobutan und dessen Additionssalze mit einer pharmazeutisch annehmbaren Säure.

**8.** Verbindung der Formel (I) nach einem der Ansprüche 1 bis 4, nämlich (-)-1-(N,N-Dimethylaminomethyl)-1-(1-hydroxycyclohex-1-yl)-5-methoxybenzocyclobutan und dessen Additionssalze mit einer pharmazeutisch annehmbaren Säure.

**9.** Verfahren zur Herstellung der Verbindungen der Formel (I) nach Anspruch 1, **dadurch gekennzeichnet, daß** man als Ausgangsprodukt:

*eine Verbindung der Formel (II) verwendet:*

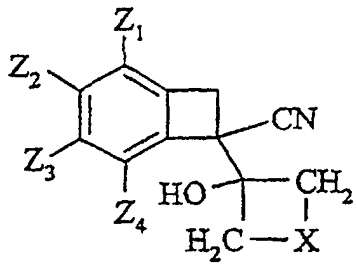

(II)

in der $Z_1$, $Z_2$, $Z_3$, $Z_4$ und X die bezüglich der Formel (I) angegebenen Bedeutungen besitzen, welche man mit einem Hydrid in Ether oder Tetrahydrofuran behandelt zur Bildung der Verbindungen der Formel (I/a), einem Sonderfall der Verbindungen der Formel (I):

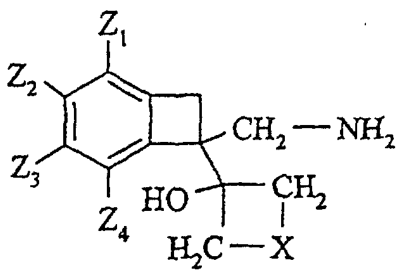

(I/a)

in der $Z_1$, $Z_2$, $Z_3$, $Z_4$ und X die oben angegebenen Bedeutungen besitzen, welche Verbindungen der Formel (I/a) man unter Anwendung klassischer Methoden der organischen Chemie, wie:

- reduzierende Aminierung ausgehend von den entsprechenden Aldehyden oder Ketonen in Gegenwart von Natriumcyanborhydrid oder von Natriumtriacetoxyborhydrid, oder
- nucleophile Substitution der Derivate der Formel (III):

$$R'_1Z \qquad \text{(III)}$$

in der $R'_1$ die oben für $R_1$ angegebene Bedeutung besitzt mit Ausnahme von Wasserstoff und Z eine austretende Gruppe darstellt, wie I, Br, Cl,

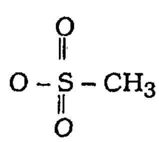

oder

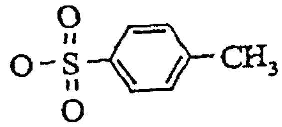

substituiert, zur Bildung der sekundären Amine der Formel (I/b), einem Sonderfall der Verbindungen der Formel (I):

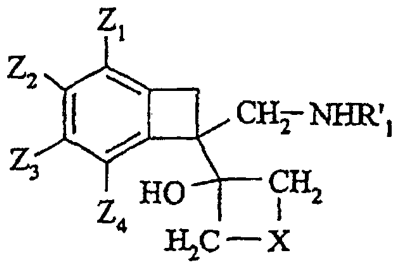

(I/b)

in der $Z_1$, $Z_2$, $Z_3$, $Z_4$, X und $R'_1$ die oben angegebenen Bedeutungen besitzen,
welche Verbindungen der Formel (I/b) erneut mit Hilfe der gleichen Methoden, wie sie oben beschrieben worden sind, substituiert werden zur Bildung der tertiären Amine der Formel (I/c), einem Sonderfall der Verbindungen der Formel (I):

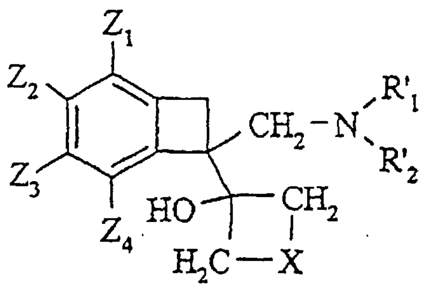

(I/c)

in der $Z_1$, $Z_2$, $Z_3$, $Z_4$ und X die oben angegebenen Bedeutungen besitzen und $R'_1$ und $R'_2$ die oben für $R_1$ bzw. $R_2$ mit Ausnahme von Wasserstoff angegebenen Bedeutungen besitzen,

wobei es sich versteht, daß, wenn $R'_1$ und $R'_2$ identisch sind und jeweils von Wasserstoff verschieden sind, man die tertiären Amine der Formel (I/c) direkt ausgehend von dem Amin (I/a) erhält, ohne Isolierung des sekundären Amins (I/b).

**10.** Verfahren zur Herstellung der Verbindungen der Formel (I) nach Anspruch 1, **dadurch gekennzeichnet, daß** man als Ausgangsprodukt:

*eine Verbindung* der Formel (IV) verwendet:

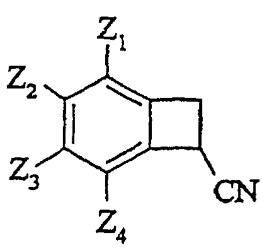

(IV)

in der $Z_1$, $Z_2$, $Z_3$ und $Z_4$ die bezüglich der Formel (I) angegebenen Bedeutungen besitzen, welche man in Gegenwart einer starken Base mit einem Amin der Formel (V) subsituiert:

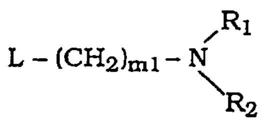

(V)

in der $R_1$ und $R_2$ die bezüglich der Formel (I) angegebenen Bedeutungen besitzen, m1 eine ganze Zahl zwischen 2 und 6 einschließlich und L eine austretende Gruppe, wie ein Halogenatom, eine Mesylatgruppe, eine Tosylatgruppe oder eine Trifluormethansulfonatgruppe bedeuten,
zur Bildung der Verbindungen der Formel (VI):

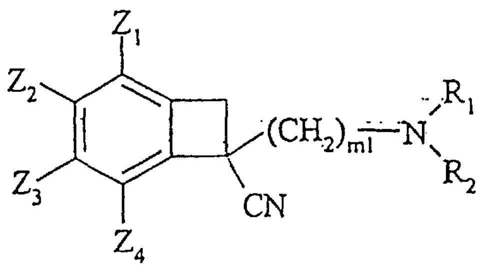

(VI)

in der $Z_1$, $Z_2$, $Z_3$, $Z_4$, $R_1$, $R_2$ und m1 die oben angegebenen Bedeutungen besitzen,
welche Verbindungen der Formel (VI) man unter den Bedingungen der sauren alkoholischen Hydrolyse in Gegenwart einer Verbindung der Formel (VII):

$$G - OH \qquad (VII)$$

in der G eine geradkettige oder verzweigte ($C_1$-$C_6$)-Alkylgruppe darstellt, behandelt zur Bildung der Verbindungen der Formel (VIII):

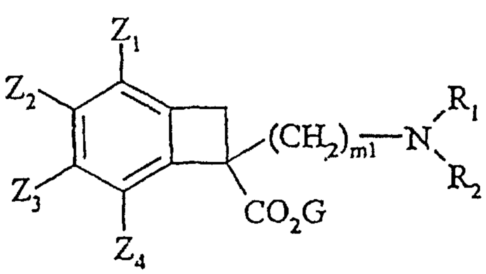

(VIII)

in der $Z_1$, $Z_2$, $Z_3$, $Z_4$, $R_1$, $R_2$, G und m1 die oben angegebenen Bedeutungen besitzen.
welche Verbindungen der Formel (VIII) man mit einer Dimagnesiumverbindung der Formel (IX):

$$Z'\text{-}Mg\text{-}CH_2\text{-}X\text{-}CH_2\text{-}Mg\text{-}Z' \qquad (IX)$$

in der X die bezüglich der Formel (I) angegebenen Bedeutungen besitzt und Z' ein Halogenatom darstellt, behandelt zur Bildung der Verbindungen der Formel (I/d), einem Sonderfall der Verbindungen der Formel (I):

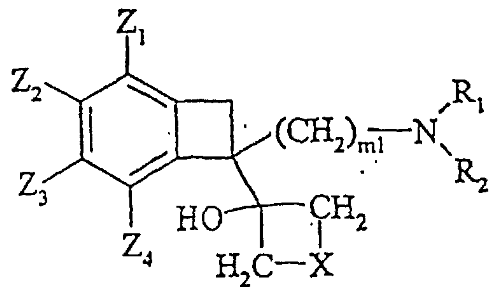

(I/d)

in der $Z_1$, $Z_2$, $Z_3$, $Z_4$, $R_1$, $R_2$, X und m1 die oben angegebenen Bedeutungen besitzen.

**11.** Verfahren nach einem der Ansprüche 9 und 10, **dadurch gekennzeichnet, daß** man die Verbindungen der Formel (I/e):

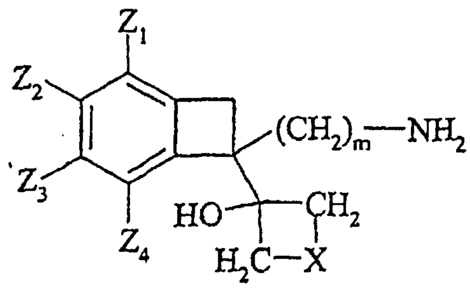

(I/e)

in der $Z_1$, $Z_2$, $Z_3$, $Z_4$, X und m die bezüglich der Formel (I) angegebenen Bedeutungen besitzen, mit *2-Chlorethyl-isocyanat* in Acetonitril und in Gegenwart von Triethylamin *behandelt* zur Bildung der Verbindungen der Formel (I/f), einem Sonderfall der Verbindungen der Formel (I):

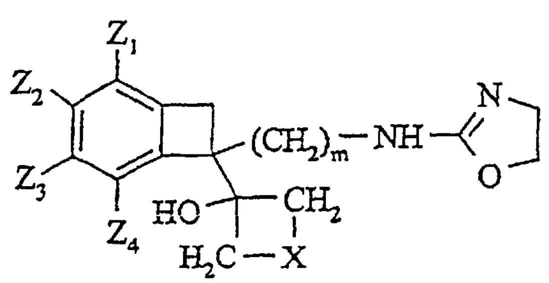

(I/f)

in der $Z_1$, $Z_2$, $Z_3$, $Z_4$, X und m die oben angegebenen Bedeutungen besitzen.

**12.** Verfahren nach einem der Ansprüche 9 und 10, **dadurch gekennzeichnet, daß** man die Verbindungen der Formel (I/e):

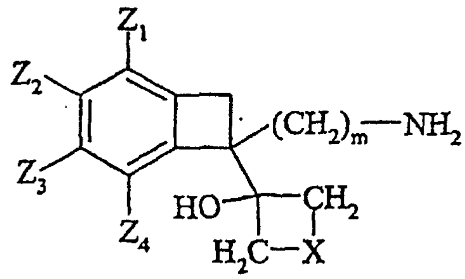

(I/e)

in der $Z_1$, $Z_2$, $Z_3$, $Z_4$, X und m die bezüglich der Formel (I) angegebenen Bedeutungen besitzen, mit *Imidazolin-2-yl-sulfonsäure* in Gegenwart einer organischen oder anorganischen Base in einem Alkohol oder in Acetonitril *behandelt* zur Bildung der Verbindungen der Formel (I/g):

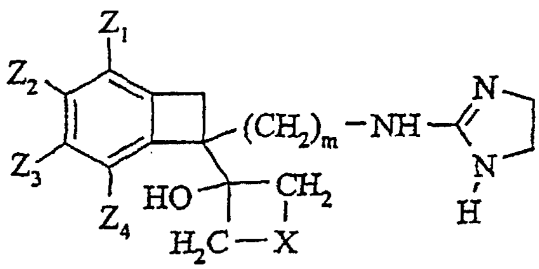

(I/g)

in der $Z_1$, $Z_2$, $Z_3$, $Z_4$, X und m die oben angegebenen Bedeutungen besitzen.

**13.** Pharmazeutische Zubereitungen enthaltend als Wirkstoff mindestens eine Verbindung der Formel (I) nach einem der Ansprüche 1 bis 8 allein oder in Kombination mit einem oder mehreren inerten, nichttoxischen, pharmazeutisch annehmbaren Trägermaterialien oder Bindemitteln.

**14.** Pharmazeutische Zubereitungen nach Anspruch 13 enthaltend mindestens einen Wirkstoff nach einem der Ansprüche 1 bis 8 zur Behandlung von Depressionen, Panikanfällen, kompulsiven Obsessionsstörungen, Phobien, impulsiven Störungen, Drogenmißbrauch und Angst.

## Claims

**1.** Compounds of formula (I) :

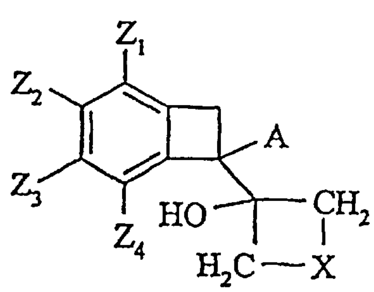

(I)

wherein :

$Z_1$, $Z_2$, $Z_3$ and $Z_4$, which may be the same or different, each represent a hydrogen atom, a halogen atom, a linear or branched $(C_1\text{-}C_6)$alkyl group, a linear or branched $(C_2\text{-}C_6)$alkenyl group, a linear or branched $(C_2\text{-}C_6)$ alkynyl group, a $[(C_3\text{-}C_8)$cycloalkyl$]\text{-}(C_1\text{-}C_6)$alkyl group in which the alkyl moiety is linear or branched, a hydroxy group, a linear or branched $(C_1\text{-}C_6)$alkoxy group, a benzyloxy group a $(C_2\text{-}C_6)$alkenoxy group in which the alkenyl moiety is linear or branched, a linear or branched $(C_2\text{-}C_6)$alkynoxy group, a cyano group, a trifluoromethyl group, a trifluoromethoxy group, a nitro group, a group of formula $-OSO_2CF_3$, $OSO_2CH_3$, $-NHCOCH_3$, $-NHCOCF_3$, $-NHSO_2CH_3$,

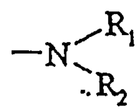

or a group of formula

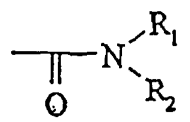

wherein
$R_1$ and $R_2$, which may be the same or different, each represent a hydrogen atom, a linear or branched ($C_1$-$C_6$) alkyl group, a linear or branched ($C_2$-$C_6$)alkenyl group, a linear or branched ($C_2$-$C_6$)alkynyl group, an aryl-($C_1$-$C_6$)alkyl group in which the alkyl moiety is linear or branched, or a [($C_3$-$C_8$)cycloalkyl]-($C_1$-$C_6$)alkyl group in which the alkyl moiety is linear or branched,
X represents

- an oxygen atom,
- a group of fomula $S(O)_p$ wherein p represents an integer from 0 to 2 inclusive.
- a group of formula $-(CH_2)_n$ wherein n represents an integer from 1 to 4 inclusive,
- or a group of formula $-CH_2$-Y-$CH_2$- wherein Y represents an oxygen atom, a selenium atom, a group >S $(O)_p$ wherein p is as defined above, >N-$R_1$ or

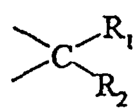

  wherein $R_1$ and $R_2$ are as defined above,

A represents a group of formula :

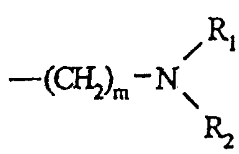

or

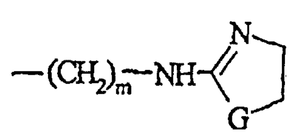

wherein
$R_1$ and $R_2$ are as defined above,

m represents an integer from 1 to 6 inclusive, and
G represents an oxygen atom or the group NH,

their isomers and addition salts thereof with a pharmaceutically acceptable acid.

**2.** Compounds of formula (I) according to claim 1 **characterised in that** A represents a group of formula

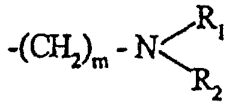

wherein m, $R_1$ and $R_2$ are as defined for formula (I), their isomers and addition salts thereof with a pharmaceutically acceptable acid.

**3.** Compounds of formula (I) according to claim 1 **characterised in that** X represents a group of formula $-(CH_2)_n-$ wherein n is as defined for formula (I), their isomers and addition salts thereof with a pharmaceutically acceptable acid.

**4.** Compounds of formula (I) according to any one of claims 1 to 3, **characterised in that**:

- A represents a group of formula

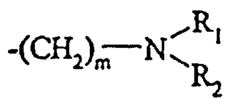

wherein $R_1$ and $R_2$ are as defined above and m is 1,
- and X represents a group of formula $-(CH_2)_n-$ wherein n is 3,

their isomers and addition salts thereof with a pharmaceutically acceptable acid.

**5.** Compounds of formula (I) according to claim 1, **characterised in that** X represents a group of formula $-CH_2-Y-CH_2-$ wherein Y is as defined for formula (I), their isomers and addition salts thereof with a pharmaceutically acceptable acid.

**6.** Compound of formula (I) according to any one of claims 1 to 4 which is 1-(N,N-dimethylaminomethyl)-1-(1-hydroxycyclohex-1-yl)-5-methoxybenzocyclobutane, and its addition salts with a pharmaceutically acceptable acid.

**7.** Compound of formula (I) according to any one of claims 1 to 4 which is 1-(N,N-dimethylaminomethyl)-1-(1-hydroxycyclohex-1-yl)-4-methoxybenzocyclobutane, and its addition salts with a pharmaceutically acceptable acid.

**8.** Compound of formula (I) according to any one of claims 1 to 4 which is (-)-1-(N,N-dimethylaminomethyl)-1-(1-hydroxycyclohex-1-yl)-5-methoxybenzocyclobutane, and its addition salts with a pharmaceutically acceptable acid.

**9.** Process for the preparation of compounds of formula (I) according to claim 1, **characterised in that** there is used as starting material :

*<u>a compound of formula (II)</u>* :

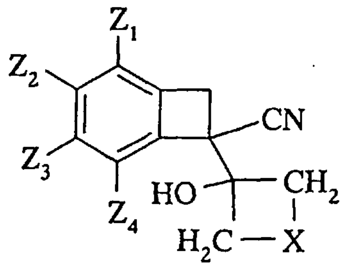

(II)

wherein $Z_1$, $Z_2$, $Z_3$, $Z_4$ and X are as defined for formula (I), which is treated with a hydride in ether or in tetrahydrofuran, to yield the compounds of formula (I/a), a particular case of the compounds of formula (I):

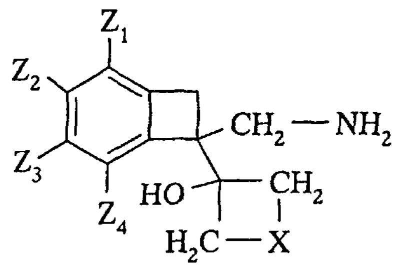

(I/a)

wherein $Z_1$, $Z_2$, $Z_3$, $Z_4$ and X are as defined above, which compounds of formula (I/a) are substituted using conventional methods of organic chemistry, such as :

- reductive amination starting from the corresponding aldehydes or ketones, in the presence of sodium cyanoborohydride or sodium triacetoxyborohydride, or
- nucleophilic substitution of compounds of formula (III) :

$$R'_1Z \qquad (III)$$

wherein $R'_1$ has the meaning given above for $R_1$ with the exception of the meaning hydrogen and Z is a leaving group, such as I, Br, Cl,

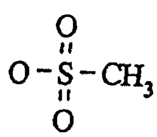

or

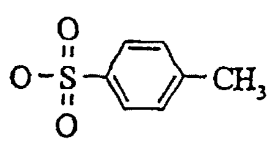

to yield the secondary amines of formula (I/b), a particular case of the compounds of formula (I) :

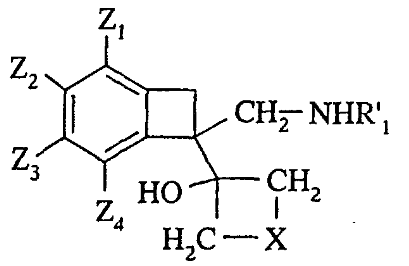

(I/b)

wherein $Z_1$, $Z_2$, $Z_3$, $Z_4$, X and $R'_1$ are as defined above,
which compounds of formula (I/b) are again substituted using the same methods as those described above to yield the tertiary amines of formula (I/c), a particular case of the compounds (I)

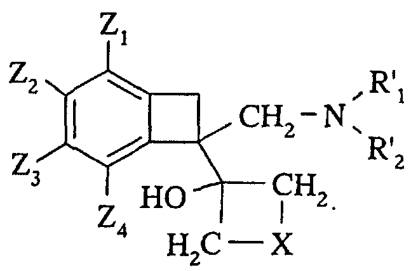

(I/c)

wherein $Z_1$, $Z_2$, $Z_3$, $Z_4$ and X are as defined above and $R'_1$ and $R'_2$ have the meanings given above for $R_1$ and $R_2$, respectively, with the exception of the meaning hydrogen,
with the proviso that when $R'_1$ and $R'_2$ are the same, each being other than hydrogen, the tertiary amines of formula (I/c) are obtained directly from the amine (I/a) without having to isolate the secondary amine (I/b).

**10.** Process for the preparation of compounds of formula (I) according to claim 1, **characterised in that** there is used as starting material:

*a compound of formula (IV)* :

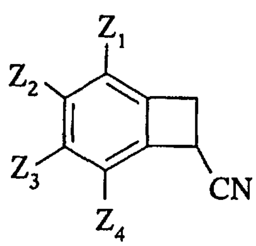

(IV)

wherein $Z_1$, $Z_2$, $Z_3$, $Z_4$ are as defined for formula (I),
which is substituted, in the presence of a strong base, by an amine of formula (V) :

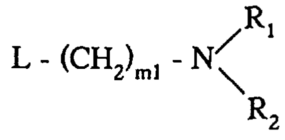

(V)

wherein $R_1$ and $R_2$ are as defined for formula (I), m1 is an integer from 2 to 6 inclusive and L represents a leaving group, such as a halogen atom, a mesylate, tosylate or trifluoromethanesulphonate group,

to yield compounds of formula (VI):

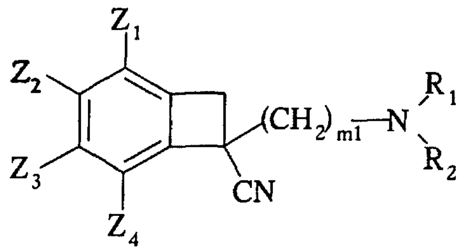

(VI)

wherein $Z_1$, $Z_2$, $Z_3$, $Z_4$, $R_1$, $R_2$ and $m_1$ are as defined above, which compounds of formula (VI) are treated under conditions of acid alcoholic hydrolysis in the presence of a compound of formula (VII) :

$$G - OH \quad (VII)$$

wherein G represents a linear or branched ($C_1$-$C_6$)alkyl group, to yield compounds of formula (VIII):

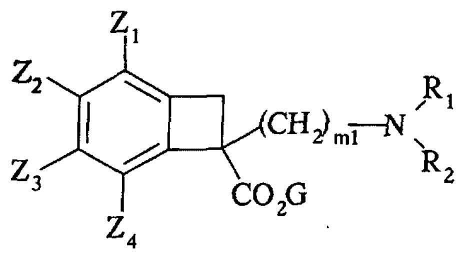

(VIII)

wherein $Z_1$, $Z_2$, $Z_3$, $Z_4$, $R_1$, $R_2$, G and m1 are as defined above, which compounds of formula (VIII) are treated with a dimagnesium compound of formula (IX) :

$$Z'\text{-}Mg\text{-}CH_2\text{-}X\text{-}CH_2\text{-}Mg\text{-}Z' \quad (IX)$$

wherein X is as defined for formula (I) and Z' represents a halogen atom, to yield the compounds of formula (I/d), a particular case of the compounds of formula (I):

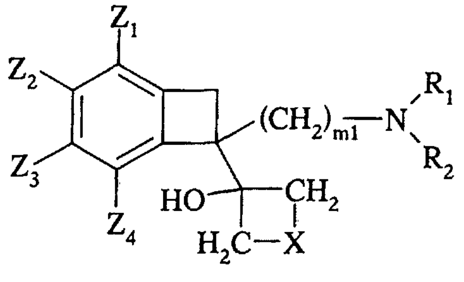

(I/d)

wherein $Z_1$, $Z_2$, $Z_3$, $Z_4$, $R_1$, $R_2$, X and m1 are as defined above.

**11.** Process according to either claim 9 or claim 10, **characterised in that** the compounds of formula (I/e):

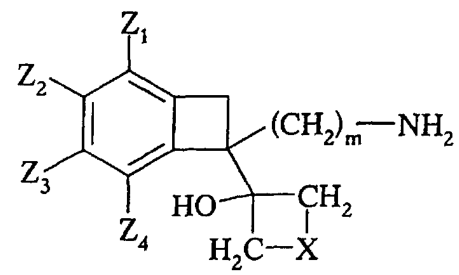

(I/e)

wherein $Z_1$, $Z_2$, $Z_3$, $Z_4$, X and m are as defined for formula (I), are treated with 2-chloroethyl isocyanate in acetonitrile in the presence of triethylamine to yield the compounds of formula (I/f), a particular case of the compounds of formula (I):

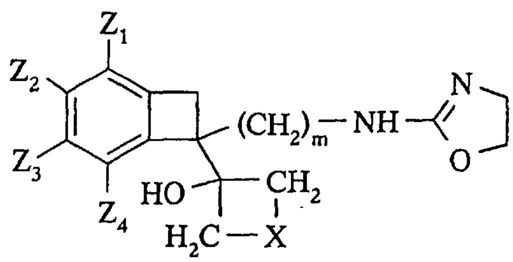

(I/f)

wherein $Z_1$, $Z_2$, $Z_3$, $Z_4$, X and m are as defined above.

12. Process according to either claim 9 or claim 10, **characterised in that** the compounds of formula (I/e):

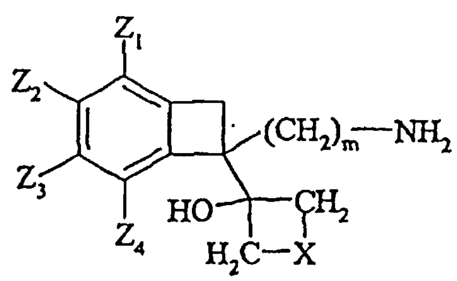

(I/e)

wherein $Z_1$, $Z_2$, $Z_3$, $Z_4$, X and m are as defined for formula (I), are treated with imidazolin-2-ylsulphonic acid in the presence of an organic or mineral base in an alcohol or in acetonitrile to yield the compounds of formula (I/g):

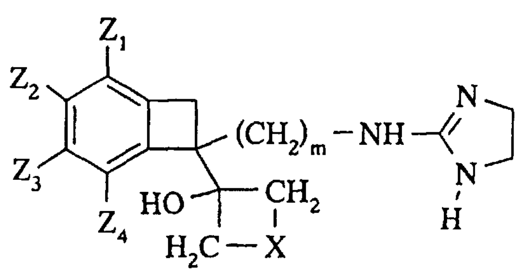

(I/g)

wherein $Z_1$, $Z_2$, $Z_3$, $Z_4$, X and m are as defined above.

13. Pharmaceutical compositions comprising as active ingredient at least one compound of formula (I) according to any one of claims 1 to 8, on its own or in combination with one or more inert, non-toxic, pharmaceutically acceptable

excipients or carriers.

**14.** Pharmaceutical compositions according to claim 13 comprising at least one active ingredient according to any one of claims 1 to 8 for use in the treatment of depression, panic attacks, obsessive compulsive disorders, phobias, impulsive disorders, drug abuse and anxiety.